Europäisches Patentamt

European Patent Office

Office européen des brevets

(19)

(11) Veröffentlichungsnummer: **0 198 236**
A2

# (12) EUROPÄISCHE PATENTANMELDUNG

(21) Anmeldenummer: **86103490.8**

(22) Anmeldetag: **14.03.86**

(51) Int. Cl.⁴: **B 01 J 31/06,** B 01 J 31/10, C 07 C 1/20

(30) Priorität: **15.03.85 DE 3509292**

(43) Veröffentlichungstag der Anmeldung: **22.10.86**
**Patentblatt 86/43**

(84) Benannte Vertragsstaaten: **DE FR GB IT**

(71) Anmelder: **Klein, Joachim, Prof. Dr., Hühnerkamp 21, D-3300 Braunschweig (DE)**
Anmelder: **Widdecke, Hartmut, Dr., Unter den Eichen 12, D-3171 Bokensdorf (DE)**
Anmelder: **Haupt, Ulrich, Hirschbergstrasse 15, D-3300 Braunschweig (DE)**

(72) Erfinder: **Klein, Joachim, Prof. Dr., Hühnerkamp 21, D-3300 Braunschweig (DE)**
Erfinder: **Widdecke, Hartmut, Dr., Unter den Eichen 12, D-3171 Bokensdorf (DE)**
Erfinder: **Haupt, Ulrich, Hirschbergstrasse 15, D-3300 Braunschweig (DE)**

(74) Vertreter: **Lins, Edgar, Dipl.-Phys. et al, Patentanwälte Gramm + Lins Theodor-Heuss-Strasse 2, D-3300 Braunschweig (DE)**

(54) **Katalysator und Verfahren zur Herstellung von reinen tertiären Olefinen.**

(57) Insbesondere zur Herstellung von reinen tertiären Olefinen durch katalytische Spaltung ihrer Alkyl-tert-alkylether bei erhöhter Temperatur wird ein Katalysator aus einem porösen Trägermaterial und polymer gebundenen Säuregruppen angegeben, bei dem das Trägermaterial ausschließlich im Oberflächenbereich, vorzugsweise im Bereich der Porenoberflächen die polymer gebundenen Säuregruppen aufweist. Ein derartiger Katalysator erlaubt eine sehr hohe Selektivität bei hohen Umsätzen.

EP 0 198 236 A2

# Patentanwälte GRAMM + LINS

Dipl.-Ing. Prof. Werner Gramm
Dipl.-Phys. Edgar Lins

European Patent Attorneys

0198236

Prof. Dr. Joachim Klein
Hühnerkamp 21
3300 Braunschweig

Dr. Hartmut Widdecke
Unter den Eichen 12
3171 Bokensdorf

Ulrich Haupt
Hirschbergstraße 15
3300 Braunschweig

Anwaltsakte
539-1 EP-1

Datum
13. März 1986

Katalysator und Verfahren zur Herstellung von
reinen tertiären Olefinen

Die Erfindung betrifft einen Katalysator bestehend aus einem
porösen Trägermaterial und polymer gebundenen Säuregruppen.
Die Erfindung betrifft ferner ein Verfahren zur Herstellung
von reinen tertiären Olefinen unter Verwendung eines Katalysators.

Alkyl-tert-alkylether werden an festen Katalysator-Systemen
gespalten, um die entsprechenden tertiären Olefine mit hohem
Reinheitsgrad herzustellen, wie sie zur Herstellung von Polymeren und höherwertigen Chemikalien benötigt werden. Der hierbei anfallende Alkohol und der nicht umgesetzte Alkyl-tert-
alkylether werden vom Olefin abgetrennt und in die der Spaltung vorgeschalteten Synthesestufe zurückgeführt.

Aus Gründen der Einfachheit und Übersichtlichkeit wird im
folgenden auf die industriell wichtige Spaltung von Methyl-
tert-butylether (MTBE) Bezug genommen, obwohl das beschriebene Verfahren auch auf alle anderen Spaltungen von Alkyl-
tert-alkylether anwendbar ist.

- 2 -

Ein wesentlicher Nachteil der bisher bekannten sauren Katalysatorsysteme zur Durchführung der Alkyl-tert-alkylether-Spaltung am Beispiel der MTBE-Spaltung liegt darin, daß bei Umsätzen von über 80 % leicht unerwünschte Nebenreaktionen ablaufen, wie die Bildung von Dimethylether aus dem erhaltenen Methanol, die Hydratisierung des erhaltenen Isobutens und das bei der Dimethylether-Bildung ebenfalls entstehende Wasser und die Oligomerisierung des Isobutens (Hydrocarbon Processing, Int.Ed., 60(8), 101 bis 106).

Insbesondere die Dimethylether-Bildung als Folgereaktion wirkt sich hierbei sehr störend aus, da die bis in den ppm-Bereich hinein nötige Abtrennung des gasförmigen Dimethylethers vom Isobuten schwierig und kostenintensiv ist. Darüber hinaus muß das in die MTBE-Synthese zurückgeführte Methanol vom Wasser, welches mit der Dimethylether-Bildung entsteht, befreit werden.

Nur mit einem großen verfahrenstechnischen Aufwand ist es möglich, mit herkömmlichen Säure-Katalysatoren die Spaltung selektiv zu betreiben, indem man die Spalt-Reaktion zusammen mit einer Destillation in einem Destillationskolonnenboden gleichzeitig ausführt (vgl. DE-A-32 10 435).

Andere Verfahren mit modifizierten anorganischen Katalysatoren verlangen zur Erzielung akzeptabler Umsätze relativ hohe Spalttemperaturen bei geringen Kontaktbelastungen. Hierbei auftretende Katalysator-Desaktivierungsprozesse erfordern aufwendige Regenerationsverfahren und Konditionierungsmaßnahmen (vgl. DE-A-29 24 869, DE-A-30 48 084, DE-A-31 24 294).

Der Erfindung liegt die Aufgabe zugrunde, einen Katalysator der eingangs erwähnten Art anzugeben, mit dem das Auftreten von unerwünschten Folgereaktionen vermindert wird und daher beispielsweise die Spaltung von Alkyl-tert-alkylethern zu tertiären Olefinen bei einem hohen Umsatz mit hoher Selekti-

- 3 -

vität und relativ niedrigen Temperaturen möglich ist.

Diese Aufgabe wird erfindungsgemäß mit einem Katalysator der eingangs erwähnten Art gelöst, bei dem das Trägermaterial die polymer gebundenen Säuregruppen ausschließlich im Oberflächenbereich aufweist.

Mit der Erfindung ist ein völlig neues Katalysatorsystem angegeben, bei dem sich ausschließlich im Bereich der Oberfläche polymer gebundene Säuregruppen auf einem Trägermaterial befinden. Erfindungsgemäß können die Säuregruppen sich entweder an der Oberfläche kleiner Trägermaterialteilchen befinden (äußere Oberfläche) oder auch im Bereich der Porenoberfläche des porösen Trägermaterials gebunden sein (innere Oberfläche).

Bevorzugt wird die Anordnung der polymer gebundenen Säuregruppen auf der Porenoberfläche (innere Oberfläche, die allerdings die äußere Oberfläche mit umfaßt), weil dadurch die Menge des benötigten Katalysatormaterials reduziert werden kann.

Der erfindungsgemäße Katalysator erzielt erhebliche Vorteile bei seiner Verwendung zur katalytischen Spaltung der Alkyl-tert-alkylether zu den tertiären Olefinen. Selbst bei Umsätzen bis über 97 % kann Alkyl-tert-alkylether hochselektiv gespalten werden. Nachgeschaltete Trennoperationen zur Aufarbeitung des Produktstroms können so einfach dimensioniert werden, und eine Edukt-Rückführung braucht nur im geringen Ausmaß stattzufinden.

Der Erfindung liegt die Erkenntnis zugrunde, daß innerhalb eines permeablen Trägermaterials angeordnete Säuregruppen die Produktselektivitäten erheblich vermindern, weil sie Folgereaktionen begünstigen. Bei dem erfindungsgemäßen Katalysator kann die katalytisch begünstig-

- 4 -

- 4 -

te Reaktion nur beim Kontakt mit der Oberfläche stattfinden, so daß die Bedingungen für Folgereaktionen ungünstig sind.

Das poröse Trägermaterial des Katalysators kann durch organische oder anorganische Matrizen gebildet sein.

Bei organischen Polymermatrizen kann die Bindung unmittelbar an der Polymermatrix erfolgen. Für die Ausbildung des Katalysators kommen insbesondere Brönsted-Säuregruppen, also Sulfonsäure oder Phosphorsäure in Frage. Bevorzugt werden Sulfonsäuregruppen verwendet, da sie besonders hohe Katalysatorbelastungen ermöglichen. Als organisches Polymer wird als Trägermaterial bevorzugt vernetztes Polystyrol verwendet.

In einem Festbettreaktor erzielt ein derartiges Katalysator-System bei der MTBE-Spaltung in Abhängigkeit von der Spalttemperatur Umsätze bis zu 98 % bei Methanol-Selektivitäten von 100 %. Die Isobuten-Selektivitäten betrugen hierbei im allgemeinen über 99 %.

Die Erfindung soll im folgenden anhand von Beispielen näher erläutert werden.

Als Alkylether-Spaltreaktor wurde ein temperierbarer Rohrreaktor mit einem Innendurchmesser von 10 mm eingesetzt. Dieser war auf eine Länge von 100 mm mit Katalysatorkörnern gefüllt. Die Katalysatorkörner bestanden aus einem porösen Trägermaterial, das durch ein zu 18 % mit Divinylbenzol vernetztes makroporöses Polystyrolharz in Kugelform gebildet war. Das Trägermaterial hatte eine Oberfläche von 110 m²/g. Die Kugeln wiesen einen Durchmesser von 0,3 bis 1,2 mm auf. Zur besseren Wärmeleitung der Katalysatorschicht wurde Edel-stahlgries als Inertmaterial hinzugefügt. Temperaturmessun-gen im Katalysator-Festbett waren axial wie auch radial möglich. Die Zudosierung des Substrats erfolgte über eine Kolbenpumpe. Die Zusammensetzung des Produktstroms wurde on-line mittels eines Gaschromatographen erfaßt.

Die folgenden Abkürzungen finden im weiteren Verwendung:

OSKAR = <u>O</u>berflächen<u>s</u>ulfonierter <u>Ka</u>talysato<u>r</u>
WHSV = "Weight-hourly-space-velocity" mit der
          Einheit (g MTBE / g Katalysator / Stunde)
MTBE = Methyl-tert-butylether
S(MeOH) = Methanol-Selektivität (%)
S(Iso) = Isobuten-Selektivität (%)
T(axial) = axiale Reaktoraustrittstemperatur (°C)

Das Einsatzprodukt war MTBE (99,9 Gew.-%), der Reaktionsdruck 1 bar.

Die Herstellung des Katalysators erfolgte mit dem angegebenen Trägermaterial. 11 g dieser Polymermatrix wurden in 175 ml Nitromethan vorgelegt und unter Rühren langsam mit 100 ml 100%iger Schwefelsäure versetzt. Nach ca. 11 h Reaktionszeit bei Raumtemperatur konnte der Katalysator OSKAR 1 und nach ca. 19 h der Katalysator OSKAR 2 abgetrennt und aufgearbeitet werden.

Die Katalysatoren waren an ihrer gesamten Oberfläche sulfoniert und es ergaben sich für die makroporösen Polymer-Matrizen die $H^+$-Austauschkapazitäten (in Milliäquivalent meq/g) von:

OSKAR 1: 0,13 meq/g
OSKAR 2: 0,21 meq/g

Unter Verwendung dieser Katalysatoren ergaben sich für verschiedene Reaktionstemperaturen und verschiedene Durchströmgeschwindigkeiten die folgenden Werte:

- 6 -

Beispiel 1:

Katalysator: OSKAR 3; Kapazität: 0,13 meq $H^+$/g
Reaktionstemperatur: 123°C

| W H S V: (1/h) | Umsatz: (%) | S(MeOH): (%) | S(Iso): (%) | T(axial): (°C) |
|---|---|---|---|---|
| 11,8 | 77,8 | 100 | 100 | 115 |
| 8,8 | 82,8 | 100 | 100 | 120 |
| 7,4 | 84,6 | 100 | 99,7 | 121 |
| 5,9 | 85,7 | 100 | 99,3 | 123 |
| 4,4 | 85,9 | 100 | 99,1 | 123 |
| 2,9 | 86,3 | 100 | 96,2 | 123 |
| 1,5 | 88,1 | 92,6 | 82,2 | 123 |

Beispiel 2:

Katalysator: OSKAR 3; Kapazität: 0,13 meq $H^+$/g
Reaktionstemperatur: 142°C

| W H S V: (1/h) | Umsatz: (%) | S(MeOH): (%) | S(Iso): (%) | T(axial): (°C) |
|---|---|---|---|---|
| 23,5 | 79,0 | 100 | 100 | 121 |
| 20,6 | 82,1 | 100 | 99,7 | 124 |
| 17,7 | 86,1 | 100 | 99,7 | 127 |
| 14,7 | 90,3 | 100 | 99,5 | 133 |
| 11,8 | 92,1 | 100 | 99,4 | 138 |
| 8,8 | 93,0 | 100 | 98,8 | 141 |
| 7,4 | 93,1 | 100 | 98,7 | 142 |
| 5,9 | 93,3 | 100 | 97,2 | 142 |
| 4,4 | 93,4 | 100 | 95,9 | 142 |
| 2,9 | 93,9 | 99,0 | 89,0 | 142 |

Beispiel 3:

Katalysator: OSKAR 3; Kapazität: 0,13 meq $H^+$/g
                Reaktionstemperatur: 162°C

| W H S V: (1/h) | Umsatz: (%) | S(MeOH): (%) | S(Iso): (%) | T(axial): (°C) |
|---|---|---|---|---|
| 29,4 | 83,2 | 100 | 100 | 133 |
| 26,5 | 86,9 | 100 | 100 | 136 |
| 23,5 | 90,0 | 100 | 100 | 140 |
| 20,6 | 93,1 | 100 | 100 | 145 |
| 17,7 | 95,2 | 100 | 100 | 151 |
| 14,7 | 96,3 | 100 | 99,7 | 157 |
| 11,8 | 96,8 | 100 | 99,4 | 160 |
| 8,8 | 97,0 | 100 | 98,9 | 162 |
| 5,9 | 97,0 | 100 | 97,5 | 162 |
| 2,9 | 97,4 | 97,8 | 89,3 | 162 |

Beispiel 4:

Katalysator: OSKAR 4; Kapazität: 0,21 meq $H^+$/g
                Reaktionstemperatur: 119°C

| W H S V: (1/h) | Umsatz: (%) | S(MeOH): (%) | S(Iso): (%) | T(axial): (°C) |
|---|---|---|---|---|
| 10,2 | 80,4 | 100 | 99,7 | 111 |
| 8,9 | 83,0 | 100 | 99,5 | 114 |
| 7,7 | 84,4 | 100 | 99,3 | 116 |
| 6,4 | 85,1 | 100 | 99,0 | 118 |
| 5,1 | 85,4 | 100 | 98,6 | 118 |
| 3,8 | 85,6 | 100 | 97,0 | 119 |
| 2,6 | 86,5 | 99,0 | 90,3 | 119 |

- 8 -

Beispiel 5:

Katalysator: OSKAR 4  Kapazität: 0,21 meq $H^+$/g
Reaktionstemperatur: 138°C

| W H S V: (1/h) | Umsatz: (%) | S(MeOH) (%) | S(Iso): (%) | T(axial): (°C) |
|---|---|---|---|---|
| 20,4 | 83,1 | 100 | 99,5 | 114 |
| 17,9 | 86,5 | 100 | 99,4 | 117 |
| 15,3 | 89,2 | 100 | 99,3 | 122 |
| 12,8 | 91,4 | 100 | 98,8 | 129 |
| 10,2 | 92,6 | 100 | 98,2 | 134 |
| 7,7 | 93,2 | 100 | 96,3 | 137 |
| 5,1 | 93,7 | 100 | 93,1 | 138 |
| 2,6 | 94,8 | 96,3 | 75,4 | 138 |

Beispiel 6:

Katalysator: OSKAR 4; Kapazität: 0,21 meq $H^+$/g
Reaktionstemperatur: 157°C

| W H S V: (1/h) | Umsatz: (%) | S(MeOH): (%) | S(Iso): (%) | T(axial): (°C) |
|---|---|---|---|---|
| 28,1 | 86,5 | 100 | 100 | 122 |
| 25,5 | 88,5 | 100 | 99,7 | 124 |
| 23,0 | 90,8 | 100 | 99,7 | 128 |
| 20,4 | 92,7 | 100 | 99,6 | 132 |
| 17,9 | 94,4 | 100 | 99,5 | 139 |
| 15,3 | 95,6 | 100 | 98,9 | 145 |
| 12,8 | 96,3 | 100 | 98,5 | 152 |
| 10,2 | 96,8 | 100 | 97,6 | 156 |
| 7,7 | 97,0 | 100 | 96,5 | 157 |
| 5,1 | 97,2 | 99,1 | 91,7 | 157 |
| 2,6 | 97,8 | 94,3 | 75,1 | 157 |

- 9 -

Beispiel 7: (Vergleich)

Ein herkömmlicher sulfonsaurer, makroporöser Ionenaustauscher, der ebenfalls auf einer mit 18% DVB vernetzten Polystyrol-Matrix basiert, wurde zum Vergleich bei der MTBE-Spaltung eingesetzt.

Deutliche Unterschiede hinsichtlich der Produkt-Selektivitäten werden in diesem Zusammenhang sichtbar. Insbesondere die MeOH-Selektivität ist deutlich schlechter. Bereits bei Umsätzen im Bereich von 80-90% sind Dimethylether-Anteile in Prozentgröße feststellbar.

Katalysator: Lewatit SPC 118  (Hersteller: BAYER AG) Kapazität: 4,7 meq $H^+$/g

Reaktionstemperatur: 138°C

| W H S V (1/h) | Umsatz: (%) | S(MeOH): (%) | S(Iso): (%) | T(axial): (°C) |
|---|---|---|---|---|
| 51,5 | 78,2 | 100 | 98,1 | 109 |
| 46,4 | 82,0 | 100 | 97,4 | 113 |
| 41,2 | 84,6 | 100 | 96,8 | 116 |
| 36,1 | 87,3 | 99,3 | 94,7 | 120 |
| 30,9 | 90,2 | 98,7 | 92,3 | 125 |
| 25,8 | 92,7 | 97,6 | 87,5 | 132 |
| 20,6 | 95,0 | 94,8 | 76,2 | 138 |
| 15,5 | 96,3 | 87,9 | 59,8 | 141 |
| 10,3 | 97,7 | 72,5 | 43,5 | 139 |

Im Gegensatz hierzu zeigt der oben beschriebene oberflächensulfonierte Katalysator erst bei deutlich höheren Umsätzen MeOH-Verluste, diese aber auch erst dann, wenn bei den thermodynamisch maximal möglichen MTBE-Umsätzen vorher die Isobuten-Selektivität merklich gesunken ist.

- 10 -

Die ausreichenden Selektivitäten der herkömmlichen Katalysatoren bei Teilumsätzen kann zur Einsparung des erfindungsgemäßen Katalysatormaterials dazu verwendet werden, einen
Reaktor stromaufwärts mit herkömmlichem Katalysatormaterial
zu füllen, um damit einen Teilumsatz durchzuführen und das
erfindungsgemäße Katalysatormaterial für die Erzielung des
Endumsatzes anschließen zu lassen.

Li/bk

0198236

Dipl.-Ing. Prof. Werner Gramm
Dipl.-Phys. Edgar Lins

European Patent Attorneys

Prof. Dr. Joachim Klein
Hühnerkamp 21
3300 Braunschweig

Dr. Hartmut Widdecke
Unter den Eichen 12
3171 Bokensdorf

Ulrich Haupt
Hirschbergstraße 15
3300 Braunschweig

Anwaltsakte
539-1 EP-1

Datum
13. März 1986

Patentansprüche:

1. Katalysator bestehend aus einem porösen Trägermaterial und polymer gebundenen Säuregruppen, dadurch gekennzeichnet, daß das Trägermaterial ausschließlich im Oberflächenbereich die polymer gebundenen Säuregruppen aufweist.

2. Katalysator nach Anspruch 1, dadurch gekennzeichnet, daß das Trägermaterial die polymer gebundenen Säuregruppen ausschließlich im Bereich der Porenoberflächen des porösen Trägermaterials aufweist.

3. Katalysator nach Anspruch 1 oder 2, dadurch gekennzeichnet, daß die Säuregruppen Brönsted-Säuregruppen sind.

4. Katalysator nach Anspruch 3, dadurch gekennzeichnet, daß die Säuregruppen Sulfonsäuregruppen sind.

5. Katalysator nach einem der Ansprüche 1 bis 4, dadurch gekennzeichnet, daß das Trägermaterial durch poröse Polymermatrizen gebildet ist und daß die Säuregruppen nur im Bereich der Porenoberfläche mit Austauschkapazitäten von bis zu 0,6 Milliäquivalent $H^+$/g Polymer-Matrix gebunden sind.

- 2 -

Theodor-Heuss-Straße 2
D-3300 Braunschweig    Bundesrepublik Deutschland

Telefon              0531-80079
Telex                0952620 gramm d
Telegrammadresse     Patent Braunschweig

- 2 -

6. Katalysator nach einem der Ansprüche 1 bis 5, gekennzeichnet durch eine spezifische Oberfläche von 10 bis 200 m²/g.

7. Katalysator nach Anspruch 5, gekennzeichnet durch eine spezifische Oberfläche von 50 bis 150 m²/g.

8. Katalysator nach einem der Ansprüche 1 bis 7, dadurch gekennzeichnet, daß er in einem Festbettreaktor angeordnet ist, in dem stromaufwärts ein herkömmlicher Katalysator vorgeschaltet ist.

9. Katalysator nach einem der Ansprüche 1 bis 8, dadurch gekennzeichnet, daß das Trägermaterial ein organisches Polymer, vorzugsweise ein vernetztes Polystyrol ist.

10. Verfahren zur Herstellung von reinen tertiären Olefinen durch katalytische Spaltung ihrer Alkyl-tert-alkylether bei erhöhter Temperatur, gekennzeichnet durch die Verwendung eines Katalysators nach einem der Ansprüche 1 bis 9.

11. Verfahren nach Anspruch 10, dadurch gekennzeichnet, daß die Kontaktbelastung des Katalysators zwischen 0,1 und 100 g Substanz pro g Katalysator und h eingestellt wird.


Patentanwälte
G r a m m    +    L i n s
Li/bk